# EUROPÄISCHE PATENTSCHRIFT

(11) **EP 1 587 434 B1**
(45) Veröffentlichungstag und Bekanntmachung des Hinweises auf die Patenterteilung: **17.01.2007**
(21) Anmeldenummer: 04764864.7
(22) Anmeldetag: 06.09.2004
(51) Int. Cl.: A61B 17/32

(54) **MEDIZINISCHES INSTRUMENT ZUM PRÄPARIEREN VON GEWEBE**
MEDICAL INSTRUMENT FOR DISSECTING TISSUE
INSTRUMENT MEDICAL POUR LA DISSECTION DE TISSUS

(30) Priorität: 05.09.2003 DE 10342002
(43) Veröffentlichungstag der Anmeldung: 26.10.2005
(73) Patentinhaber: Karl Storz GmbH & Co. KG, 78532 Tuttlingen (DE)
(72) Erfinder: STAMMBERGER, Heinz, AT-8020 Graz (AT)
(74) Vertreter: Heuckeroth, Volker
(86) Internationale Anmeldenummer: PCT/EP2004/009915
(87) Internationale Veröffentlichungsnummer: WO 2005/023122

(56) Entgegenhaltungen:
- GB-A- 2 198 950
- US-A- 5 582 618
- US-A1- 2002 143 354
- US-B1- 6 500 189

## Beschreibung

Die vorliegende Erfindung betrifft ein medizinisches Instrument zum Präparieren von menschlichem und/oder tierischem Gewebe, mit einem Schaft, mit einem ersten Werkzeug und einem zweiten Werkzeug an einem distalen Ende des Schafts, wobei das erste Werkzeug und das zweite Werkzeug durch eine axiale Relativbewegung zueinander in der Art einer Stanze zusammenwirken, und wobei zumindest eines der Werkzeuge ein Schneideelement aufweist, das so angeordnet ist, dass es mit dem anderen Werkzeug einen Stanzbereich bildet, der eine Ebene definiert, die mit einer Längsachse des Schafts im Bereich des distalen Endes einen Winkel von ungleich 0° bildet, wobei sich das distalseitige Werkzeug in seinem distalen Bereich zum distalen Ende hin in Längsrichtung des Schafts konvex im Durchmesser verjüngt.

Ein solches Instrument ist aus GB-A-2 198 950 bekannt.

Bei dem Instrument zum Präparieren von Gewebe handelt es sich um ein Instrument zum Abtrennen von Gewebe, bspw. Hartgewebe und/oder Knochen, insbesondere handelt es sich dabei um eine Gewebestanze.

Derartige chirurgische Instrumente werden im Rahmen der minimalinvasiven Chirurgie dazu verwendet, Hartgewebe oder Knochen im menschlichen oder tierischen Körper, üblicherweise unter endoskopischer Sichtkontrolle abzutrennen. Dazu weisen derartige Instrumente einen langerstreckten Schaft auf, an dessen distalem Ende zumindest ein bewegliches Werkzeug angeordnet ist, das mit einem weiteren beweglichen oder unbeweglichen Werkzeug am distalen Ende des Schafts zum Abtrennen des Gewebes zusammenwirkt.

Chirurgische Instrumente, deren Werkzeuge auf Basis einer axialen Relativbewegung miteinander zusammenwirken, haben den Vorteil, dass beim Öffnen und Schließen der Werkzeuge sich der effektive Durchmesser des Instruments nicht ändert. Dadurch wird eine Schädigung des umliegenden Gewebes oder umliegender Knochen durch ein Öffnen und Schließen der Werkzeuge verhindert.

Dadurch, dass es beim Öffnen und Schließen der Werkzeuge nicht zu einer Vergrößerung des effektiven Durchmessers des Instruments kommt, können solche Instrumente auch in kleineren Körperöffnungen eingesetzt werden als Instrumente, die zangenartig öffnende Werkzeuge aufweisen.

Solche Gewebestanzen werden daher bevorzugt in der Hals-, Nasen-, Ohren-Chirurgie (HNO-Chirurgie) eingesetzt, in der der kleinere effektive Durchmesser des Instruments von großem vorteil ist.

Es sind ferner Instrumente unter den Bestellnummern 651050, 651055, 651060 oder 651065 aus dem DE-Katalog "Endoskope und Instrumente für HNO", 6. Ausgabe, 1/2000, Seite SI-FESS 11, der Karl Storz GmbH & Co. KG bekannt.

Solche Instrumente weisen einen Schaft auf, an dessen distalem Ende zwei Werkzeuge angeordnet sind. Von diesen beiden Werkzeugen ist zumindest eines axial relativ zu dem zweiten Werkzeug verschiebbar. Zumindest eines dieser zwei Werkzeuge weist bei dem bekannten Instrument an der dem zweiten Werkzeug zugewandten Seite ein etwa kreisförmiges Schneideelement auf. Durch die axiale Relativbewegung untereinander wirken die beiden Werkzeuge in Form einer Stanze zusammen. Hierbei wird durch das zumindest eine Schneideelement ein Stanzbereich definiert, der etwa in rechtem Winkel zur Längsachse des Schaftes in dessen distalem Bereich steht.

Beim Einsatz dieser Instrumente hat sich allerdings gezeigt, dass insbesondere beim Durchtrennen von Knochenlamellen, die gegenüber dem Stanzbereich abgewinkelt sind, es zu hohen Belastungen in kleinen Bereichen der Schneideelemente des Instruments kommt. Es hat sich hierbei ergeben, dass dieses zu einer Beschädigung der Schnittflächen bis hin zu einem Abbrechen von Metallstücken aus den Schneideelementen führen kann. Diese herausgebrochenen Metallteile sind extrem scharfkantig und verbleiben nach dem Herausbrechen im Körper des Patienten. Diese zurückgebliebenen Metallstücke können durch das Gewebe wandern und dort nicht unbeträchtlichen Schaden anrichten. Dies ist vor allen Dingen im Bereich der HNO-Chirurgie höchst gefährlich für den Patienten.

Dadurch ist die Betriebssicherheit der bekannten Instrumente nicht gewährleistet.

Aus der Patentschrift US-A-5 582 618 ist ein chirurgisches Instrument bekannt, das einen Schaft aufweist, an dessen distalem Ende zwei Werkzeuge angeordnet sind, von denen eines axial relativ zum zweiten Werkzeug verschiebbar ist. Diese beiden Werkzeuge weisen je an einer oberen Kante ein gerades Schneideelement auf. Durch diese Schneideelemente wird eine gerade Stanzlinie definiert.

Der Nachteil dieser Instrumente liegt darin, dass mit diesen nur ein Schneiden entlang der geraden Stanzlinie möglich ist. Ein Schneiden über einen Teilkreis, wie es mit den oben beschriebenen Instrumenten möglich ist und wie es vor allen Dingen in der HNO-Chirurgie wünschenswert ist, ist mit einem solchen Instrument nicht möglich.

Das aus GB-A-2 198 950 bekannte Instrument weist ein distalseitiges Werkzeug auf, das eine nach proximal gerichtete kreisförmige schneidkante aufweist, die mit einer Schneidkante des proximalen Werkzeugs schneidend zusammenwirkt. Das distale Werkzeug ist an seinem distalen Ende zunächst ohne Durchmesserverjüngung parallel zur Längsachse des Schafts geradlinig ausgebildet, und geht dann in eine halbkugelige Form am äußersten distalen Ende über.

Die Dokumente US-A-6 500 189 und US-A-2002/143354 offenbaren eine Gewebestanze, bei der das distalseitige Werkzeug sich von proximal her zunächst in Richtung distales Ende konvex verjüngt und dann in eine sich konisch verjüngende Spitze übergeht.

Zwischen der konischen Spitze und dem konvexen Abschnitt ist ein konkav ausgebildeter Übergang vorhanden.

Wenn ein Instrument der eingangs genannten Art, wie dies ohne Beschränkung der Allgemeinheit für das Instrument der vorliegenden Erfindung der Fall ist, zum Stanzen von Knochenlamellen verwendet werden soll, ist es erforderlich, das Instrument mit den distalseitigen Werkzeugen zunächst durch die Knochenlamelle durchzustoßen, bis der Stanzbereich im Bereich der Knochenlamelle zu liegen kommt. Die bisher bekannten Instrumente sind für das Durchtrennen von Knochenlamellen nicht geeignet.

Es ist daher Aufgabe der vorliegenden Erfindung, ein chirurgisches Instrument der eingangs genannten Art anzugeben, das hinsichtlich seiner Eignung zum Stanzen von Knochenlamellen verbessert ist.

Hinsichtlich des eingangs genannten medizinischen Instruments wird die Aufgabe dadurch gelöst, dass sich die Außenkonturlinien des distalseitigen Werkzeugs im Längsmittelschnitt gesehen am distalen Ende unter einem Winkel α von kleiner als 160° schneiden, und dass das proximalseitige Werkzeug einen geringeren Außendurchmesser aufweist, als ein proximales Ende des distalseitigen Werkzeuges, so dass beim Stanzen das proximalseitige Werkzeug in das distalseitige Werkzeug eintaucht.

Mit dieser Ausgestaltung des distalseitigen Werkzeuges des erfindungsgemäßen Instruments eignet sich das Instrument insbesondere zum Stanzen von Knochenlamellen, weil das Instrument mit seinem distalseitigen Werkzeug voran zunächst die Knochenlamelle durchstoßen kann, bis der weiter proximal liegende Stanzbereich der beiden Werkzeuge an der Knochenlamelle zu liegen kommt. Das Durchstoßen der Knochenlamellen wird dadurch erleichtert, weil das distalseitige Werkzeug, das die Knochenlamelle durchstoßen muss, am distalen Ende spitz zuläuft. Mit dieser Geometrie des distalseitigen Werkzeugs wird ein unerwünschtes Abbrechen der Knochenlamelle an einer anderen als der zu stanzenden Stelle vermieden.

Das proximalseitige Werkzeug weist einen geringeren Außendurchmesser auf als ein proximales Ende des distalseitigen Werkzeugs, so dass beim Stanzen das proximalseitige Werkzeug in das distalseitige Werkzeug eintaucht.

Auch diese Maßnahme ist besonders vorteilhaft, wenn vor dem Stanzen das distalseitige Werkzeug durch das zu stanzende Gewebe durchgestoßen werden muss. Durch den geringeren Durchmesser des proximalseitigen werkzeuges stellt dieses beim Vorschieben des Instruments durch das zu durchstoßende Gewebe kein Hindernis dar, an dem sich das Gewebe verfangen kann. Vielmehr gleitet dann das Gewebe über das proximale Ende des distalseitigen Werkzeugs hinaus, so dass die beiden Werkzeuge mühelos in die zum Stanzen erforderliche Position bezüglich des Gewebes gebracht werden können.

In bevorzugten Ausgestaltungen ist der zuvor genannte Winkel kleiner als 140°, insbesondere kleiner als 120° und weiter insbesondere kleiner als 100°, vorzugsweise aber größer als 50°.

Während es möglich ist, dass das äußerste distale Ende des distalseitigen Werkzeugs zwar noch relativ spitz ist, jedoch senkrecht zur Längsachse des Schafts abgeschnitten oder abgerundet ist, ist es weiterhin bevorzugt, wenn die Außenkonturlinien zu einer Spitze zusammenlaufen.

Der Vorteil hierbei besteht darin, dass durch die punktförmige Spitze das distalseitige Werkzeug vor dem Durchstoßen der Knochenlamelle punktgenau auf die Knochenlamelle aufgesetzt werden kann, das distalseitige Werkzeug somit als Visierpunkt für das Instrument dient. Die zuvor genannte Spitze kann auch vorzugsweise mit einer Rundung mit einem sehr kleinen Radius versehen sein, um ungewollte Traumatisierungen an Gewebe zu vermeiden.

In einer weiter bevorzugten Ausgestaltung geht der konvexe Abschnitt des distalseitigen Werkzeugs in einen zumindest annähernd parallel zur Längsachse des Schafts verlaufenden geraden Abschnitt über.

Hierbei ist von Vorteil, dass eine übermäßige Durchmesservergrößerung des distalseitigen Werkzeugs in seinem proximalen Bereich vermieden wird, so dass die Werkzeuge insgesamt im Durchmesser weiterhin sehr schmalbauend ausgestaltet bleiben, wobei dennoch ein möglichst großer Stanzbereich durch die beiden Werkzeuge geschaffen wird.

In einer weiter bevorzugten Ausgestaltung ist der Winkel zwischen der Längsachse des Schafts im Bereich des distalen Endes des Schafts und der durch den Stanzbereich definierten Ebene ungleich 90°.

Es hat sich gezeigt, dass durch ein Abwinkeln des Stanzbereichs gegenüber der Längsachse des Schafts eine größere Kontaktfläche zwischen den Schneideelementen und den zu durchtrennenden Knochenlamellen geschaffen wird. Durch ein Vergrößern dieser Kontaktfläche wird die Belastung der Schneideelemente der Werkzeuge bezogen auf die Fläche verringert. Dadurch können Knochenlamellen deutlich besser durchtrennt werden und es kommt zu deutlich weniger Verschleiß- und Ermüdungserscheinungen an dem medizinischen Instrument. Die Betriebssicherheit wird auf diese Weise verbessert, da die Gefahr eines Abbrechens von werkzeugteilen verringert wird.

In einer weiteren bevorzugten Ausführungsform liegt der Winkel zwischen der Längsachse des Schafts im Bereich des distalen Endes des Schafts und der durch den Stanzbereich definierten Ebene zwischen etwa 20° und etwa 80° und noch bevorzugter zwischen etwa 30° und etwa 70°.

Es hat sich gezeigt, dass die Mehrzahl der zu durchtrennenden Knochenlamellen, vor allem im Bereich der HNO-Chirurgie, in etwa diesem Winkel zur Längsachse des Schafts des medizinischen Instruments angetroffen werden. Besonders häufig werden Knochenlamellen mit einem Winkel von etwa 45° angetroffen, im Bereich der Stirnhöhle werden allerdings auch Winkel von etwa 65° angetroffen.

Je mehr der Winkel zwischen der durch den Stanzbereich definierten Ebene und der Längsachse des distalen Endes des Schafts dem Verlauf der Knochenlamelle angepasst ist, desto größer ist die Auflagefläche der Schneideelemente an der Knochenlamelle und umso geringer ist die Belastung auf diese Schneideelemente.

In weiteren einer bevorzugten Ausführungsform verläuft die durch den Stanzbereich definierte Ebene entweder von oben-proximal nach unten-distal, von oben-distal nach unten-proximal, von rechts-proximal nach links-distal oder von rechts-distal nach links-proximal.

Diese verschiedenen Ausrichtungen des Stanzbereichs in den vier Raumrichtungen sind vor allem für Instrumente wichtig, die im distalen Abschnitt des Schaftes eine Krümmung aufweisen, da bei diesen eine Ausrichtung des Stanzbereichs durch einfaches Verdrehen des Schafts nicht möglich oder erschwert ist.

Die Ausrichtung des Stanzbereichs in eine der vier Raumrichtungen erleichtert außerdem die Verwendung eines medizinischen Instruments gemäß der Erfindung deutlich, da dem Operateur die Ausrichtung des Stanzbereichs im Verhältnis zu einem am proximalen Ende des Schafts angebrachten Griffteils leicht erkenntlich ist.

In einer weiteren bevorzugten Ausführungsform ist das Schneideelement vollumfänglich am ersten oder zweiten Werkzeug ausgebildet.

Diese Ausführungsform hat den Vorteil, dass der volle Umfang der Werkzeuge zur Durchführung eines Schnitts verwendet werden kann, ohne dass dazu der Schaft verdreht werden muss. Hierdurch ist die Handhabung des Instruments verbessert. Dies ist besonders für Instrumente wichtig, die im distalen Abschnitt des Schafts eine Krümmung aufweisen, da bei diesen eine Ausrichtung des Stanzbereichs durch einfaches Verdrehen des Schafts nicht möglich oder erschwert ist.

In einer weiteren bevorzugten Ausführungsform weisen die Werkzeuge einen etwa kreisförmigen Querschnitt auf.

Ein etwa kreisförmiger Querschnitt der Werkzeuge ist vorteilhaft, da die Werkzeuge dadurch keine Ecken und Kanten aufweisen, die sowohl beim Einführen oder Entnehmen als auch während des Eingriffs selbst ungewollte Schädigung an Knochen oder an den Geweben des Patienten verursachen können.

Ein etwa kreisförmiger Querschnitt hat außerdem den Vorteil, dass ein ellipsenförmiges oder kreisförmiges Schneideelement vollumfänglich an dem Werkzeug ausgebildet werden kann.

In einer weiteren bevorzugten Ausführungsform liegt der Durchmesser der Werkzeuge im Bereich von etwa 2 mm bis etwa 8 mm, besonders bevorzugt im Bereich von etwa 3 mm bis etwa 5 mm.

Diese oben genannten Größen eignen sich vorteilhafterweise für eine Verwendung der Instrumente in der minimalinvasiven Chirurgie, insbesondere in der minimalinvasiven HNO-Chirurgie.

In einer weiteren bevorzugten Ausführungsform bilden das erste und das zweite Werkzeug im Schließzustand ein kugelförmiges oder allgemein doppelparaboloid- bzw. ellipsoidförmiges, insbesondere eiförmiges, tropfenförmiges oder keulenförmiges Profil.

Es hat sich gezeigt, dass die oben beschriebenen Profile für die Werkzeuge das Einführen des Instruments an den Operationsort deutlich erleichtern. Durch das abgerundete, sich verjüngende distale Ende der Werkzeuganordnung ist es vor allen Dingen erleichtert, das Instrument durch kleine Öffnungen hindurchzuführen. Durch die Verjüngung an der proximalen Seite der Werkzeuge wird wiederum das Entfernen des Instruments aus der Körperhöhle deutlich erleichtert.

In einer weiteren bevorzugten Ausführungsform ist das erste Werkzeug beweglich und das zweite Werkzeug unbeweglich, wobei das zweite Werkzeug distalseitig des ersten Werkzeugs angeordnet ist.

Durch diese Anordnung verschiebt sich das distale Ende des Instruments während des Stanzvorgangs nicht, was sowohl die Positionierung der Werkzeuge erleichtert, als auch die Gefahr einer Schädigung umliegenden Gewebes durch das sich bewegende distale Ende des Instruments beim Betätigen der Werkzeuge vermindert.

In einer weiteren bevorzugten Ausführungsform ist das erste Werkzeug relativ zum zweiten Werkzeug verdrehgesichert.

Diese Ausgestaltung ist besonders vorteilhaft, da dadurch sichergestellt wird, dass die Schneideelemente der beiden Werkzeuge stets optimal zusammenwirken können, was insbesondere bei einer ellipsenförmigen Ausgestaltung der Schneideelemente wichtig ist, da in diesem Fall eine Rotationssymmetrie der Schneiden fehlt.

In einer weiteren bevorzugten Ausführungsform weisen beide Werkzeuge Schneideelemente auf.

Das Anbringen von Schneideelementen an beiden Werkzeugen führt zu einem deutlich effizienteren Schneiden, im Vergleich zu einem Instrument, bei dem nur ein Werkzeug ein Schneideelement aufweist und das andere als "Amboss" der Stanze wirkt.

In einer weiteren bevorzugten Ausführungsform weist der Schaft zumindest eine Krümmung auf.

Dies ist insbesondere bei operativen Eingriffen im HNO-Bereich vorteilhaft, um die Werkzeuge am distalen Ende des Schafts an schwer zugängliche Stellen, z.B. Nischen der Stirn- oder Kieferhöhle, heranführen zu können.

In einer weiteren bevorzugten Ausführungsform weist die Krümmung des Schafts einen Krümmungswinkel im Bereich von etwa 20° bis etwa 90° und bevorzugterweise im Bereich von etwa 45° bis etwa 65° auf.

Diese Winkel haben sich als besonders geeignet herausgestellt, die oben genannten schwer zugänglichen Bereiche bei der HNO-Chirurgie zu erreichen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:
- Fig. 1: ein Ausführungsbeispiel eines erfindungsgemäßen medizinischen Instruments mit den Werkzeugen in Offenstellung in einer teilweise geschnittenen Seitenansicht;
- Fig. 2: das medizinische Instrument in Fig. 1 mit den Werkzeugen in Schließstellung in einer teilweise geschnittenen Seitenansicht, wobei die Werkzeuge vergrößert dargestellt sind;
- Fig. 3: ein weiteres Ausführungsbeispiel eines erfindungsgemäßen medizinischen Instruments mit den Werkzeugen in Offenstellung in einer teilweise geschnittenen Seitenansicht;
- Fig. 4: eine vergrößerte Darstellung der Werkzeuge des Instruments in Fig. 1 in einer teilweise geschnittenen Seitenansicht;
- Fig. 5: eine vergrößerte Darstellung der Werkzeuge des Instruments in Fig. 2 in einer teilweise geschnittenen Seitenansicht;
- Fig. 6: eine vergrößerte Darstellung der Werkzeuge des Instruments in Fig. 1 beim Schneiden einer Knochenlamelle;
- Fig. 7: eine vergrößerte Darstellung der Werkzeuge eines medizinischen Instruments nach dem Stand der Technik beim Schneiden einer Knochenlamelle;
- Fig. 8: die gleiche Darstellung wie in Fig. 6, wobei die Werkzeuge vor dem Stanzen an der Knochenlamelle anliegen;
- Fig. 9: die gleiche Darstellung wie in Fig. 7, wobei die Werkzeuge vor dem Stanzen an der Knochenlamelle anliegen;
- Fig. 10: ein noch weiteres Ausführungsbeispiel von Werkzeugen in einer vergrößerten Darstellung, die bei dem Instrument in Fig. 1 oder in Fig. 2 anstelle der dort dargestellten Werkzeuge verwendet werden können, in Seitenansicht; und
- Fig. 11: ein noch weiteres Ausführungsbeispiel von Werkzeugen zur Verwendung bei dem Instrument in Fig. 1 oder dem Instrument in Fig. 2 in vergrößerter Darstellung und in Seitenansicht.

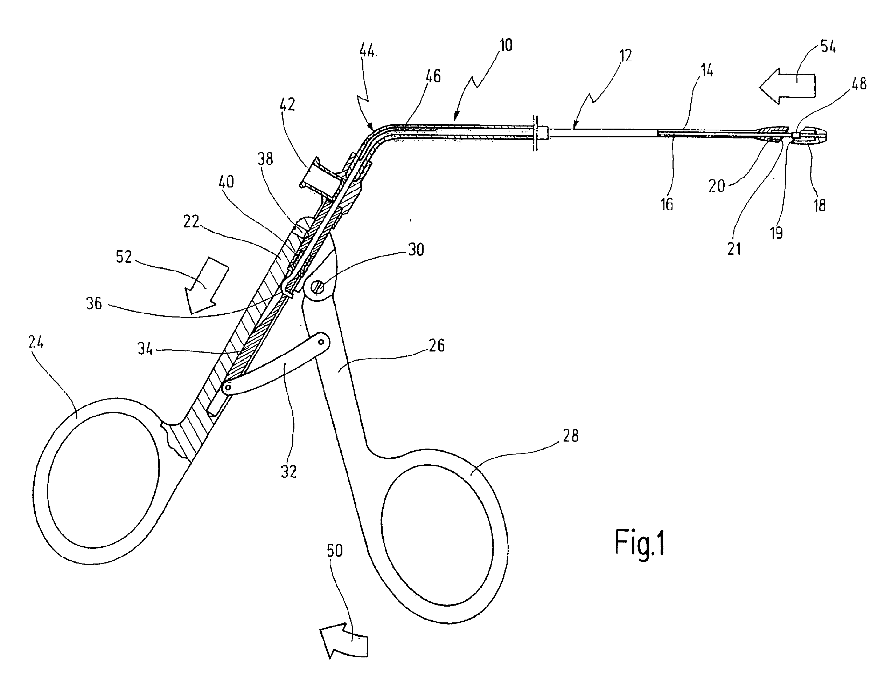

In Fig. 1 ist ein mit dem allgemeinen Bezugszeichen 10 versehenes medizinisches Instrument zum Präparieren von menschlichem und/oder tierischem Gewebe dargestellt.

Das erfindungsgemäße Instrument 10 weist einen Schaft 12 auf. Der Schaft 12 ist durch ein rohrförmiges Element 14 gebildet, in dem ein Kraftübertragungselement 16 verläuft. Das Kraftübertragungselement 16 weist hierbei über den Großteil seiner Länge einen Querschnitt in Form eines Dreiecks auf. Dadurch werden zwischen dem Kraftübertragungselement 16 und dem rohrförmigen Element 14 Spülkanäle für eine Spülflüssigkeit gebildet.

Am distalen Ende des Schafts 12 ist ein erstes Werkzeug 18 angeordnet, das an seinem proximalen Ende ein ellipsenförmiges vollumfängliches Schneideelement 19 aufweist. Das erste Werkzeug 18 ist fest mit dem Kraftübertragungselement 16 verbunden, zum Beispiel durch Laserschweißen, und relativ zum Schaft 12 axial beweglich. Proximal des ersten Werkzeugs 18 ist am distalen Ende des Schafts 12 ein zweites Werkzeug 20 angeordnet, das an seinem distalen Ende ein ellipsenförmiges vollumfängliches Schneideelement 21 aufweist. Das zweite Werkzeug 20 ist dabei unbeweglich mit dem rohrförmigen Element 14 verbunden.

Die Schneideelemente 19 und 21 sind so angeordnet, dass beim Schließen der Werkzeuge das Schneideelement 19 außen an dem Schneideelement 21 vorbeiläuft und das Schneideelement 21 in das Werkzeug 18 eintaucht.

Am proximalen Ende des Schafts 12 ist ein erstes Griffteil 22 angeordnet.

Dieses Griffteil 22 ist in Form eines Scherengriffteils ausgebildet und weist einen Ring 24 auf, der zum Durchstecken des Daumens dient.

An dem ersten Griffteil 22 ist ein bewegliches Griffteil 26 angebracht.

Dieses bewegliche Griffteil 26 ist ebenfalls als Scherengriffteil ausgebildet und weist einen Ring zum Hindurchstecken eines Fingers, z.B. des Zeigefingers oder des Mittelfingers, auf.

Das bewegliche Griffteil 26 ist mit Hilfe eines Drehgelenks 30 um eine Achse, die senkrecht zur Ebene der Griffteile 22, 26 verläuft, verdrehbar mit dem ersten Griffteil 22 verbunden.

Zwischen dem ersten Griffteil 22 und dem beweglichen Griffteil 26 ist außerdem ein Hebel 32 angebracht. Dieser Hebel 32 ist auf der einen Seite um eine Achse, die senkrecht zur Ebene der Griffteile 22, 26 verläuft, gelenkig mit dem beweglichen Griffteil 26 verbunden. Auf der anderen Seite des Hebels 32 ist dieser mit einem Schlitten 34 verbunden, der verschiebbar in dem ersten Griffteil 22 angeordnet ist. Die Verbindung zwischen dem Hebel 32 und dem Schlitten 34 ist ebenfalls als Drehgelenk ausgebildet.

In den Schlitten 34 ist das als Haken 36 ausgebildete proximale Ende des Kraftübertragungselements 16 eingehängt. Damit sind das bewegliche Griffteil 26, der Hebel 32 und der Schlitten 34 kraftschlüssig mit dem Kraftübertragungselement 16 verbunden.

Das erste Griffteil 22 weist weiterhin eine Madenschraube 38 auf, die durch eine Bohrung in dem ersten Griffteil 22 hindurchgeht. Durch Lösen der Madenschraube 38 und Aushängen des Hakens 36 aus dem Schlitten 34 kann somit der Schaft 12 von dem ersten Griffteil 22 und dem damit verbundenen zweiten Griffteil 26 abgenommen werden. Dadurch wird die Reinigung und Sterilisation des Instruments erleichtert.

Der Schaft 12 weist zusätzlich im Bereich des Hakens 36 eine Dichtung 40 auf. Durch die Bohrung der Dichtung 40 geht das proximale Ende des Kraftübertragungselements 16 bündig hindurch.

Die Dichtung 40 verhindert ein Auslaufen von Flüssigkeit wie z.B. einer Spülflüssigkeit am proximalen Ende des Schafts 12.

Im proximalen Bereich des Schafts 12 weist dieser des Weiteren ein Anschlussstück 42 für einen Spülanschluss auf. Dieser Anschluss kann dazu verwendet werden, einen Spülschlauch anzuschließen, um Spülflüssigkeit in den Schaft einzuleiten, die auf Grund der im Querschnitt dreieckigen Form des Kraftübertragungselements bis zum distalen Ende des Schafts geleitet werden kann, wo sie dann in das Operationsgebiet austreten kann.

Des Weiteren weist der Schaft 12 im proximalen Bereich eine Krümmung 44 auf.

Im Bereich dieser Krümmung 44 ist das Kraftübertragungselement 16 abgeflacht, um mit dem Kraftübertragungselement 16 eine Kraftübertragung über diese Biegung hinweg zu ermöglichen.

Das Kraftübertragungselement 16 weist an seinem distalen Ende einen Anschlag 48 auf, der beim Schließen der Werkzeuge 18, 20, deren axiale Relativbewegung begrenzt.

Nachfolgend wird die Funktion des Instruments 10 näher beschrieben.

Wird das bewegliche Griffteil 26 in Richtung des Pfeils 50 geschwenkt, bewegt der Hebel 32 den Schlitten 34 in Richtung des Rings 24, also in Richtung des Pfeils 53. Auf diese Weise wird eine Kraft auf das Kraftübertragungselement 16 ausgeübt, die ebenfalls in Richtung des Pfeils 52 wirkt. Diese Kraft wird mit Hilfe des abgeflachten Abschnitts 46 durch die Biegung 44 des Schafts 12 auf das bewegliche Werkzeug 18 übertragen. Dadurch wird das bewegliche Werkzeug 18 axial in proximale Richtung, also in Richtung des Pfeils 54, gezogen und die Werkzeuge 18, 20 dabei geschlossen, um den Schneide- oder Stanzvorgang auszuführen.

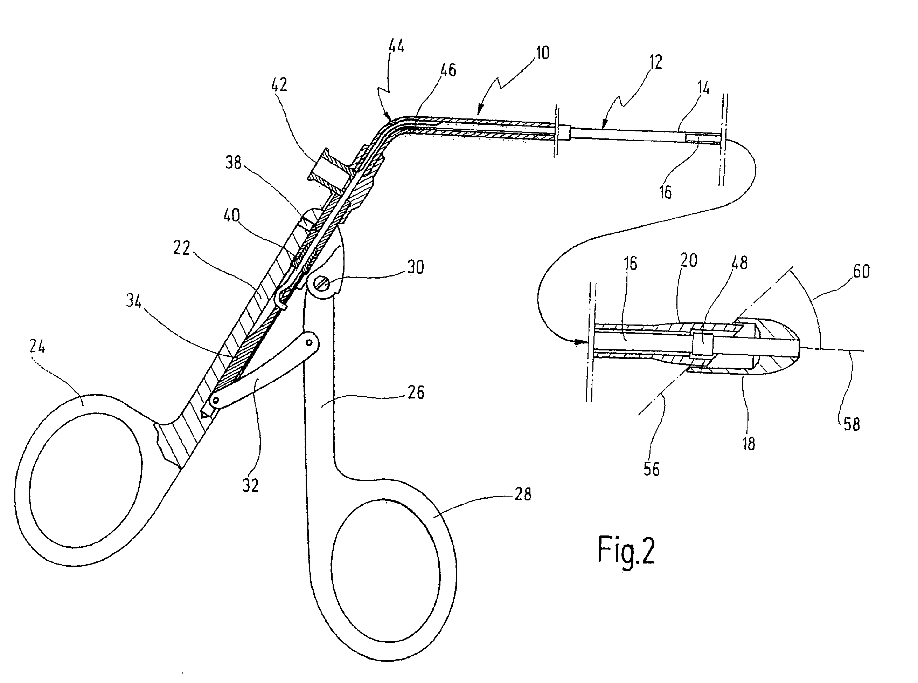

Fig. 2 zeigt das medizinische Instrument 10 nach der in Fig. 1 angedeuteten Bewegung, d.h. mit geschlossenen Werkzeugen 18, 20. Es ist hierbei ersichtlich, dass das bewegliche Griffteil 26 auf das erste Griffteil 22 zubewegt wurde, wodurch sowohl der Hebel 32 als auch der Schlitten 34 auf den Ring 24 zubewegt wurden. Diese Zugbewegung wurde durch das Kraftübertragungselement 16 auf das erste Werkzeug 18 übertragen, wodurch dieses auf das zweite Werkzeug 20 zubewegt wurde. Die Werkzeuge befinden sich somit im Schließzustand.

Hierbei ist ersichtlich, wie der Anschlag 48 gegen eine Schulter in dem zweiten Werkzeug 20 läuft.

Ebenfalls in dieser Zeichnung ist die durch den Stanzbereich definierte Ebene 56 als strichpunktierte Ebene dargestellt. Die Ebene 56 steht hierbei orthogonal zur Zeichenebene. Ein Winkel 60 zwischen der Ebene 56 und der Längsachse 58 des Schafts 12 in dessen distalem Abschnitt beträgt etwa 45°.

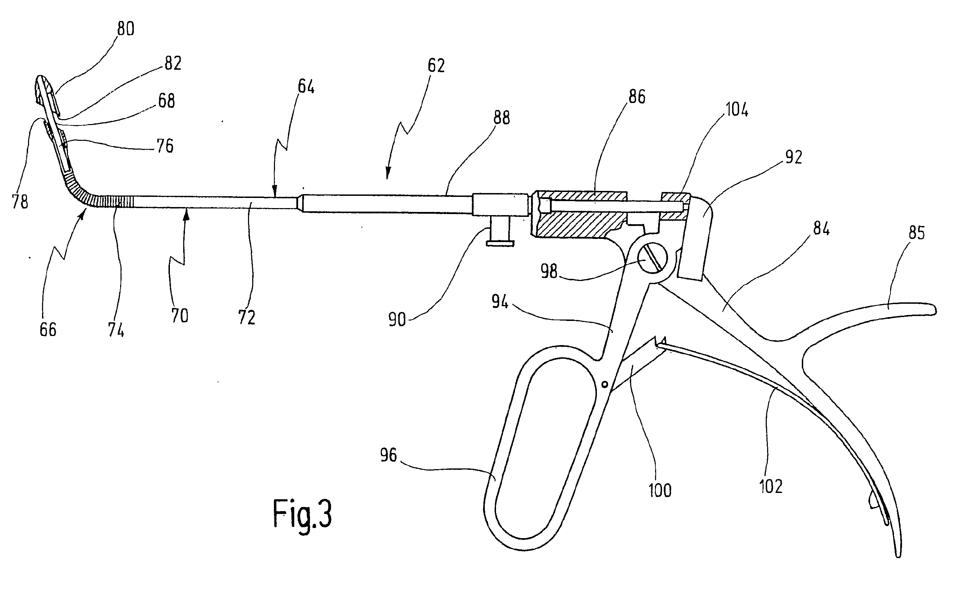

In Fig. 3 ist ein zweites Ausführungsbeispiel für ein medizinisches Instrument allgemein mit der Bezugsziffer 62 versehen.

Das Instrument 62 weist einen Schaft 64 auf, der in einem distalen Abschnitt eine Krümmung 66 aufweist.

Der Schaft 64 ist zumindest teilweise als Stab 68 ausgebildet, der in diesem Ausführungsbeispiel massiv ausgeführt ist. Weiterhin weist der Schaft 64 ein Kraftübertragungselement 70 auf, das im proximalen Bereich als Rohr 72 und im Bereich der Krümmung 66 als Schraubenfeder 74 ausgebildet ist. Die Schraubenfeder 74 und das Rohr 72 sind fest miteinander verbunden.

Distal der Schraubenfeder 74 ist ein erstes Werkzeug 76 an dem Kraftübertragungselement 70 angebracht, wobei das erste Werkzeug 76 gegenüber dem Schaft 64 axial beweglich ist. An dem distalen Ende des ersten Werkzeugs 76 ist ein Schneideelement 78 angebracht. Distal von dem ersten Werkzeug 76 ist ein zweites Werkzeug 80 angebracht, das an seinem proximalen Ende ein Schneideelement 82 aufweist. Das zweite Werkzeug 80 ist hierbei fest mit dem Stab 68 verbunden.

Das erste Werkzeug 76 und das zweite Werkzeug 80 sind hierbei so angeordnet, dass bei einer axialen Bewegung des ersten Werkzeugs 76 in Richtung des zweiten Werkzeugs 80 das Schneideelement 78 an der Innenseite des Schneideelements 82 vorbeiläuft, wobei das Schneideelement 78 des ersten Werkzeugs 76 in das zweite Werkzeug 80 eintaucht.

Am proximalen Ende des Schafts 64 ist ein erstes Griffteil 84 angeordnet.

Dieses Griffteil 84 weist einen Fortsatz 85 auf, der während der Benutzung des Instruments 62 in der Mulde zwischen Daumen und Zeigefinger aufliegt und das Griffteil dort während der Benutzung abstützt.

An seinem oberen Ende weist das erste Griffteil 84 ein rohrförmiges Element 86 auf, das unbeweglich mit dem Griffteil verbunden ist.

Das Rohr 72 und der Stab 68 gehen jeweils beweglich durch die Bohrung des rohrförmigen Elements 86 in axialer Richtung hindurch.

Das rohrförmige Element 86 wird axial zum Schaft 64 in distaler Richtung durch ein rohrförmiges Element 88 fortgesetzt. Das Rohr 72 und die Stange 68 gehen durch das rohrförmige Element 88 ebenfalls beweglich hindurch. Am proximalen Ende des rohrförmigen Elements 88 ist ein Anschlussstück 90 für einen Spülanschluss angebracht. Damit kann zwischen die Stange 68 und das Rohr 72 Spülflüssigkeit eingeführt werden.

Am proximalen Ende des Schafts 64 ist eine Halterung 92 angeordnet, die unbeweglich mit der Stange 68 verbunden ist. Diese Halterung 92 ist ebenfalls unbeweglich mit dem ersten Griffteil 84 verbunden.

Mit dem ersten Griffteil 84 ist weiterhin ein zweites Griffteil 94 verbunden, das einen Langring 96 aufweist, der geeignet ist, zum Bedienen des Instruments 62 ein, zwei oder drei Finger hindurchzustecken.

Die beiden Griffelemente 84 und 94 sind über ein Drehgelenk 98 um eine Achse, die senkrecht zur Ebene der Griffteile 84, 92 verläuft, gelenkig miteinander verbunden.

Das bewegliche Griffteil 94 weist weiterhin einen Hebel 100 auf, der mit einer Blattfeder 102 zusammenwirkt, die an dem unbeweglichen Griffteil 84 angebracht ist. Der Hebel 100 und die Blattfeder 102 wirken in einer Weise zusammen, dass die Werkzeuge 76 und 80, wenn keine Kraft auf das Instrument 62 wirkt, in offenem Zustand gehalten werden.

An seinem oberen Ende weist das bewegliche Griffteil 94 einen Reiter 104 auf. Dieser Reiter 104 ist unbeweglich mit dem Rohr 70 verbunden. Die Stange 68 geht beweglich durch den Reiter 104 hindurch.

Nachfolgend wird die Funktion des Instruments 62 näher beschrieben.

Wird das bewegliche Griffteil 94 auf das erste Griffteil 84 zubewegt, wird der Reiter 104 von der Halterung 92 wegbewegt. Dadurch wird das Rohr 72, die Schraubenfeder 74 und damit zusammenhängend das erste Werkzeug 78 axial in distaler Richtung bewegt. Gleichzeitig wird die Stange 68 und damit verbunden das zweite Werkzeug 80 durch die Halterung 92 an dem ersten Griffteil 84 zurückgehalten. Durch diese Bewegung werden die Werkzeuge geschlossen und der Schneide-/Stanzvorgang durchgeführt.

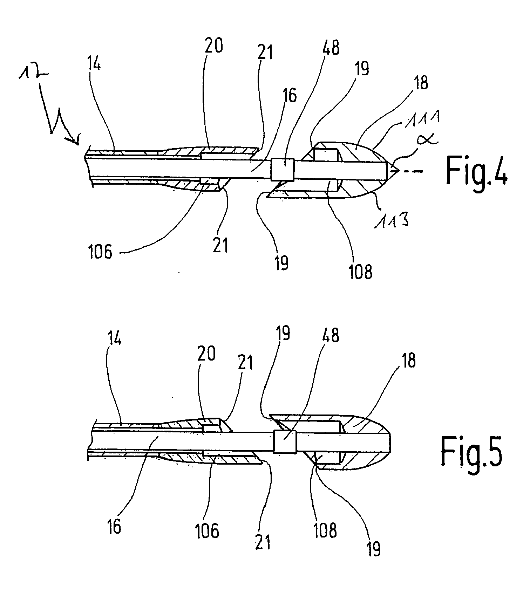

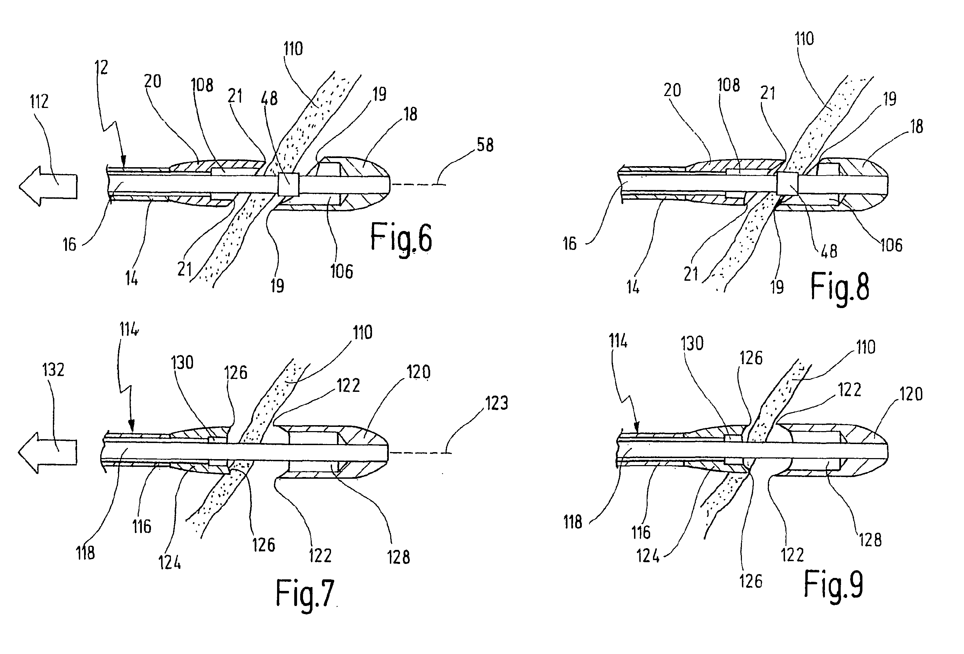

Figuren 4 und 5 zeigen eine vergrößerte Darstellung des distalen Abschnitts bzw. der Werkzeuge 18, 20 der Instrumente 10 bzw. 62. Die Darstellungen unterscheiden sich dadurch, dass in Fig. 4 die durch den Stanzbereich definierte Ebene 56 von oben-distal nach unten-proximal verläuft, während in Fig. 5 die durch den Stanzbereich definierte Ebene 56 von oben-proximal nach unten-distal verläuft.

In Fig. 4 ist ferner dargestellt, dass sich das distalseitige Werkzeug 18 zum distalen Ende hin in Längsrichtung des Schafts 12 im Durchmesser konvex verjüngt, und zwar derart, dass die Außenkonturlinien des distalseitigen Werkzeugs im Längsmittelschnitt gesehen am distalen Ende sich unter einem Winkel α von kleiner als 160° und größer als 50° schneiden. Der Winkel α beträgt bei dem Werkzeug 18 in Fig. 4 etwa 85°. Das distalseitige Werkzeug 18 endet jedoch nicht in einer Spitze, sondern bevor sich die Außenkonturlinien 111 und 113 schneiden, ist das distale Ende des Werkzeugs 18 senkrecht zur Längsachse des Schafts 12 abgeschnitten. Wegen der miniaturisierten Ausgestaltung des distalseitigen Werkzeugs 18 mit einem Durchmesser von weniger als 3 mm, ist das distale Ende des Werkzeugs 18 immer noch relativ spitz.

Weiterhin ist zu sehen, dass das bewegliche Werkzeug 18 einen Hohlraum 106 aufweist, der dazu dient, abgeschnittene Gewebe-und Knochenstücke einzuschließen, so dass diese so aus dem Operationsbereich entfernt werden können.

Das zweite Werkzeug 20 weist ebenfalls einen Hohlraum 108 auf. Dieser Hohlraum 108 dient zur Aufnahme des Anschlags 48, der die axiale Relativbewegung der Werkzeuge 18, 20 zueinander begrenzt.

Ebenfalls in diesen Darstellungen erkennbar ist das in seiner Gesamtheit keulen- bzw. paraboloidförmige Profil der Werkzeuge 18 und 20.

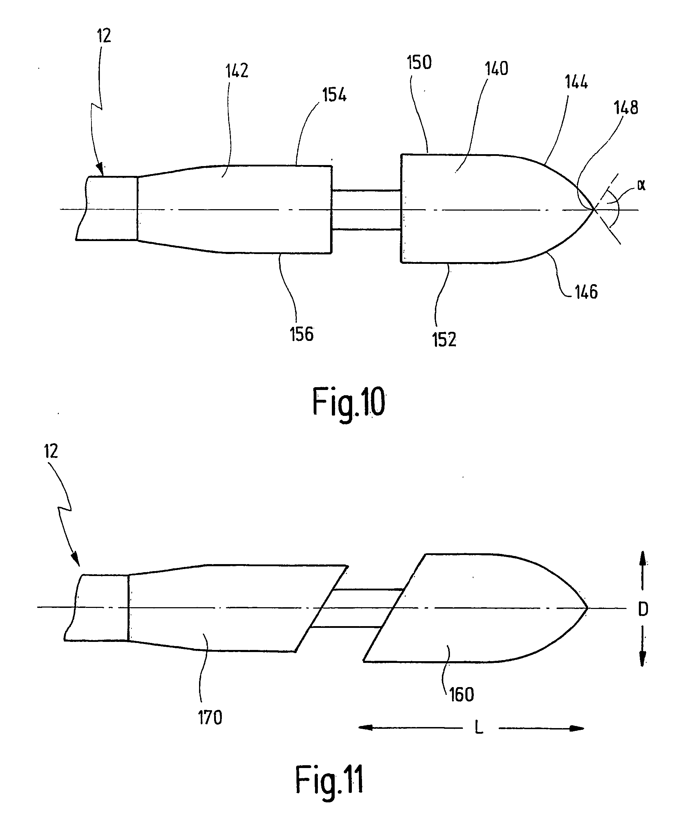

In Figuren 10 und 11 sind weitere Ausführungsbeispiele von Werkzeugen dargestellt, die anstelle der Werkzeuge 18, 20 bzw. 76, 80 bei den Instrumenten 10 oder 62 verwendet werden können.

Fig. 10 zeigt ein erstes, distalseitiges Werkzeug 140 und ein zweites, distalseitiges Werkzeug 142, das sich von den vorhergehenden Ausführungsbeispielen durch folgende Merkmale unterscheidet. Das distalseitige Werkzeug 140 weist einen sich zum distalen Ende hin in Längsrichtung des in Fig. 10 nur teilweise dargestellten Schafts 12 konvex verjüngenden Abschnitt auf. Außenkonturlinien 144 und 146 dieses konvexen Abschnitts schneiden sich wieder unter einem Winkel α, der kleiner als 160° und größer als 50° ist, wobei der Winkel α in Fig. 10 etwa 120° beträgt. Im Unterschied zu dem Werkzeug 18 laufen die Außenkonturlinien 144, 146 des Werkzeugs 140 zu einer distalen Spitze 148, die ggf. mit einem sehr kleinen Radius, beispielsweise von etwa 0,1 bis 0,5 mm, abgerundet sein kann, aus, mit der das Werkzeug 140 punktgenau auf eine beispielsweise zu durchstoßende Knochenlamelle oder ein zu durchstoßendes Gewebe aufgesetzt werden kann.

An dem konvexen Abschnitt des Werkzeugs 140 schließt sich ein parallel zur Längsachse des Schafts 12 verlaufender geradliniger Abschnitt an, dessen Außenkonturlinien 150, 152 also gerade verlaufen. Das proximalseitige Werkzeug 142 weist entsprechend distalseitig einen geraden Abschnitt mit geraden Außenkonturlinien 154, 156 auf, und weist einen geringfügig kleineren Außendurchmesser als der Innendurchmesser des proximalen Bereichs des Werkzeugs 140 auf, so dass das proximalseitige Werkzeug 142 beim Stanzen durch eine axiale Relativbewegung zu dem distalseitigen Werkzeug 140 in letzteres eintauchen kann, um Gewebe abzutrennen.

Die in Fig. 11 dargestellten Werkzeuge 160 und 170 entsprechen den Werkzeugen 140 und 142, wobei jedoch die Werkzeuge 160 und 170 einen bezüglich der Längsachse des Schafts 12 nicht senkrechten, sondern schrägen Stanzbereich bilden, wie bereits oben im Detail beschrieben wurde.

Die Länge L des distalseitigen Werkzeugs 160, das Gleiche gilt für das Werkzeug 140 in Fig. 10, ist in einem Bereich des etwa 0,7- bis 3-fachen des Durchmessers D des Werkzeuges 160 bzw. 140 bemessen.

In den Figuren 6 bis 9 ist das Schneiden einer Knochenlamelle jeweils mit dem Instrument 10 (Figuren 6 und 8) und einem Instrument nach dem Stand der Technik (Figuren 7 und 9) dargestellt.

Fig. 6 zeigt den distalen Abschnitt des Instruments 10, in einem vergrößerten Maßstab, entsprechend zu der Darstellung in Fig. 4, wobei das Instrument 10 an eine Knochenlamelle 110 herangeführt wurde, die etwa in einem Winkel von 60° zur Längsachse 58 des Schafts 12 steht.

Fig. 7 zeigt den distalen Abschnitt eines medizinischen Instruments nach dem Stand der Technik, das wiederum an die Knochenlamelle 110 herangeführt wurde. Dieses Instrument nach dem Stand der Technik weist einen Schaft 114 auf, der durch ein hülsenförmiges Element 116 gebildet wird, in dem beweglich ein Kraftübertragungselement 118 verläuft. Das Kraftübertragungselement 118 weist an seinem distalen Ende ein erstes Werkzeug 120 auf, das an seinem proximalen Ende ein etwa kreisförmiges Schneideelement 122 aufweist.

Es wird hierbei ersichtlich, dass die durch das Schneideelement 122 definierte Schnittebene senkrecht zur Längsachse 123 des Schafts 114 steht. Proximal von dem ersten Werkzeug 120 ist ein zweites Werkzeug 124 angebracht, das an seinem distalen Ende ein Schneideelement 126 aufweist. Die beiden Werkzeuge 120 und 124 weisen in ihrem Inneren je einen Hohlraum 128 und 130 zur Aufnahme von abgetrennten Hartgewebe- oder Knochenstücken auf. Die Knochenlamelle 110 steht ebenfalls in einem Winkel von etwa 60° zur Längsachse 123 des Schafts 114.

Durch ein Verschieben des jeweiligen Kraftübertragungselements 16 und 118 in proximale Richtung - jeweils in Richtung der Pfeile 131 und 132 - werden die jeweiligen ersten, axial beweglichen Werkzeuge 18 und 120 in Richtung der jeweiligen zweiten Werkzeuge 20 und 124 bewegt und die Werkzeuge 18, 20, 120 und 124 werden geschlossen. Der Vorgang des Schneidens ist hierbei jeweils in den Figuren 8 und 9 dargestellt. Die ersten Werkzeuge 18 und 120 sind hierbei axial auf die jeweiligen zweiten Werkzeuge 20 und 124 zubewegt worden und die jeweiligen Schneideelemente 19 und 21 bzw. 122 und 126 wirken auf die Knochenlamelle 110.

Es wird hieraus ersichtlich, dass bei dem Instrument 10 eine größere Anlagefläche der Schneideelemente 21 und 19 an der Knochenlamelle gegeben ist als bei dem Instrument nach dem Stand der Technik, wie in Fig. 9 dargestellt. Diese geringere Anlagefläche bei dem Instrument nach dem Stand der Technik führt häufiger zum Herausbrechen von Metallstücken, z.B. aus dem Schneideelement 122, was bei dem Instrument 10 vermieden wird.

## Patentansprüche

1. Medizinisches Instrument (10; 62) zum Präparieren von menschlichem und/oder tierischem Gewebe, mit einem Schaft (12; 68), mit einem ersten Werkzeug (18; 76; 140; 160) und einem zweiten Werkzeug (20; 80; 142; 170) an einem distalen Ende des Schafts (12; 68), wobei das erste Werkzeug (18; 76; 140; 160) und das zweite Werkzeug (20; 80; 142; 170) durch eine axiale Relativbewegung zueinander in der Art einer Stanze zusammenwirken, und wobei zumindest eines der Werkzeuge (18, 20; 76, 80; 140, 142; 160, 170) ein Schneideelement (19, 21; 78, 82) aufweist, das so angeordnet ist, dass es mit dem anderen Werkzeug (18, 20; 76, 80; 140, 142; 160, 170) einen Stanzbereich bildet, der eine Ebene (56) definiert, die mit einer Längsachse (50) des Schafts (12; 68) im Bereich des distalen Endes des Schafts (12; 68) einen Winkel (60) von ungleich 0° bildet, wobei sich das distalseitige Werkzeug (18; 76; 140; 160) in seinem distalen Bereich zum distalen Ende hin in Längsrichtung des Schafts (12; 68) konvex im Durchmesser verjüngt, **dadurch gekennzeichnet, dass** sich die Außenkonturlinien (111, 113; 144, 146) des distalseitigen Werkzeugs (18; 76; 140; 160) im Längsmittelschnitt gesehen am distalen Ende unter einem Winkel α von kleiner als 160° schneiden, und dass das proximalseitige Werkzeug (20; 80; 142; 170) einen geringeren Außendurchmesser aufweist als ein proximales Ende des distalseitigen Werkzeuges (18; 76; 140; 160), so dass beim Stanzen das proximalseitige Werkzeug (20; 80; 142; 170) in das distalseitige Werkzeug (18; 76; 140; 160) eintaucht .

2. Instrument nach Anspruch 1 , **dadurch gekennzeichnet, dass** der Winkel (α) kleiner als 140° ist.

3. Instrument nach Anspruch 1 oder 2, **dadurch gekennzeichnet, dass** der Winkel (α) kleiner als 120° ist.

4. Instrument nach einem der Ansprüche 1 bis 3, **dadurch gekennzeichnet, dass** der Winkel (α) kleiner als 100° ist.

5. Instrument nach einem der Ansprüche 1 bis 4, **dadurch gekennzeichnet, dass** die Außenkonturlinien (144, 146) zu einer Spitze (148) zusammenlaufen*

6. Instrument nach einem der Ansprüche 1 bis 5, **dadurch gekennzeichnet, dass** der konvexe Abschnitt des distalseitigen Werkzeugs (18; 76; 140; 160) zumindest annähernd in einen parallel zur Längsachse des Schafts (12) verlaufenden geraden Abschnitt übergeht.

7. Instrument nach einem der Ansprüche 1 bis 6, **dadurch gekennzeichnet, dass** der Winkel (60) ungleich 90° ist.

8. Instrument nach Anspruch 7, **dadurch gekennzeichnet, dass** der Winkel (60) zwischen der Längsachse (58) des Schafts (12; 68) im Bereich des distalen Endes des Schaft (12; 68) und der durch den Stanzbereich definierten Ebene (56) zwischen etwa 20° und etwa 80° liegt.

9. Instrument nach Anspruch 7 oder 8, **dadurch gekennzeichnet, dass** der Winkel (60) zwischen der Längsachse (58) des Schafts (12; 68) im Bereich des distalen Endes des Schafts (12; 68) und der durch den Stanzbereich definierten Ebene (56) zwischen etwa 30° und etwa 70° liegt.

10. Instrument nach einem der Ansprüche 7 bis 9, **dadurch gekennzeichnet, dass** die durch den Stanzbereich definierte Ebene (56) von oben-proximal nach unten-distal verläuft.

11. Instrument nach einem der Ansprüche 7 bis 9, **dadurch gekennzeichnet, dass** die durch den Stanzbereich definierte Ebene (56) von oben-distal nach unten-proximal verläuft.

12. Instrument nach einem der Ansprüche 7 bis 9, **dadurch gekennzeichnet, dass** die durch den Stanzbereich definierte Ebene (56) von rechts-proximal nach links-distal verläuft.

13. Instrument nach einem der Ansprüche 7 bis 9, **dadurch gekennzeichnet, dass** die durch den Stanzbereich definierte Ebene (56) von rechts-distal nach links-proximal verläuft.

14. Instrument nach einem der Ansprüche 1 bis 13, **dadurch gekennzeichnet, dass** das Schneideelement (19, 21; 78, 82) vollumfänglich am ersten oder zweiten Werkzeug (18, 20; 76, 80; 140, 142; 160, 170) ausgebildet ist.

15. Instrument nach einem der Ansprüche 1 bis 14, **dadurch gekennzeichnet, dass** die Werkzeuge (18, 20; 76, 80; 140, 142; 160, 170) einen in etwa kreisförmigen Querschnitt aufweisen.

16. Instrument nach Anspruch 15, **dadurch gekennzeichnet, dass** der Durchmesser der Werkzeuge (18, 20; 76, 80; 140, 142; 160, 170) im Bereich zwischen etwa 2 und etwa 8 mm liegt.

17. Instrument nach Anspruch 16, **dadurch gekennzeichnet, dass** der Durchmesser der Werkzeuge (18, 20; 76, 80; 140, 142; 160, 170) im Bereich zwischen etwa 3 mm und etwa 5 mm liegt.

18. Instrument nach einem der Ansprüche 1 bis 17, **dadurch gekennzeichnet, dass** das erste **Werkzeug (18;** 76) und das zweite Werkzeug (20; 80) in Schließstellung ein kugelförmiges Profil bilden.

19. Instrument nach einem der Ansprüche 1 bis 18, **dadurch gekennzeichnet, dass** das erste Werkzeug (18; 76) und das zweite Werkzeug (20; 80) in Schließstellung ein doppelparaboloid- bzw. ellipsoidförmiges Profil bilden.

20. Instrument nach einem der Ansprüche 1 bis 19, **dadurch gekennzeichnet, dass** das erste Werkzeug (18; 76) und das zweite Werkzeug (20; 80) in Schließstellung ein tropfenförmiges Profil bilden.

21. Instrument nach einem der Ansprüche 1 bis 20, **dadurch gekennzeichnet, dass** das erste Werkzeug (18; 76) und das zweite Werkzeug (20; 80) in Schließstellung ein keulenförmiges Profil bilden.

22. Instrument nach einem der Ansprüche 1 bis 21, **dadurch gekennzeichnet, dass** das eines der Werkzeuge (18, 20; 76, 80; 140, 142; 160, 170) beweglich und das zweite Werkzeug (18, 20; 76, 80; 140, 142; 160, 170) unbeweglich ist, und dass das unbewegliche Werkzeug distalseitig des beweglichen Werkzeugs angeordnet ist.

23. Instrument nach einem der Ansprüche 1 bis 22, **dadurch gekennzeichnet, dass** das erste Werkzeug (18; 76; 140; 160) relativ zum zweiten Werkzeug (20; 80; 142; 170) verdrehgesichert ist.

24. Instrument nach einem der Ansprüche 1 bis 23, **dadurch gekennzeichnet, dass** beide Werkzeuge (18, 20; 76, 80; 140, 142; 160, 170) Schneideelemente (19, 21; 78, 82) aufweisen.

25. Instrument nach einem der Ansprüche 1 bis 24, **dadurch gekennzeichnet, dass** der Schaft (12; 68) gerade ist.

26. Instrument nach einem der Ansprüche 1 bis 25, **dadurch gekennzeichnet, dass** der Schaft (12; 68) zumindest eine Krümmung (44; 66) aufweist.

27. Instrument nach Anspruch 26, **dadurch gekennzeichnet, dass** ein Krümmungswinkel der Krümmung (44; 66) im Bereich zwischen etwa 20° und etwa 90° liegt.

28. Instrument nach Anspruch 26 oder 27, **dadurch gekennzeichnet, dass** ein Krümmungswinkel der Krümmung (44; 66) im Bereich zwischen etwa 45° und etwa 65° liegt.

## Claims

1. A medical instrument (10; 62) for dissecting human and/or animal tissue, comprising a shaft (12; 68), a first tool (18; 76; 140; 160) and a second tool (20; 80; 142; 170) at a distal end of the shaft (12; 68), the first tool (18; 76; 140; 160) and the second tool (20; 80; 142; 170) cooperating in the manner of a punch by means of an axial relative movement with respect to one another, and at least one of the tools (18, 20; 76, 80; 140, 142; 160, 170) comprising a cutting element (19, 21; 78, 82) which is arranged in such a way that it forms, with the other tool (18, 20; 76, 80; 140, 142; 160, 170), a punch area that defines a plane (56) which, with a longitudinal axis (50) of the shaft (12; 68), forms an angle (60) different than 0° in the area of the distal end of the shaft (12; 68), wherein the distally arranged tool (18; 76; 140; 160) tapers in diameter, in its distal area, convexly toward the distal end in the longitudinal direction of the shaft (12; 68), **characterized in that** the external contour lines (111, 113; 144, 146) of the distally arranged tool (18; 76; 140; 160) intersect, when viewed in a section through a longitudinal central plane, at an angle a smaller than 160° at the distal end, and that the proximally arranged tool (20; 80; 142; 170) has a smaller external diameter than a proximal end of the distally arranged tool (18; 76; 140; 160) such that the proximally arranged tool (20; 80; 142; 170) engages into the distally arranged tool (18; 76; 140; 160) during punching.

2. The instrument of claim 1, **characterized in that** the angle (a) is smaller than 140°.

3. The instrument of claim 1 or 2, **characterized in that** the angle (a) is smaller than 120°.

4. The instrument of anyone of claims 1 through 3, **characterized in that** the angle (a) is smaller than 100°.

5. The instrument of anyone of claims 1 through 4, **characterized in that** the external contour lines (144, 146) run together to a point (148).

6. The instrument of anyone of claims 1 through 5, **characterized in that** the convex portion of the distally arranged tool (18; 76; 140; 160) merges at least approximately into a straight portion extending parallel to the longitudinal axis of the shaft (12).

7. The instrument of anyone of claims 1 through 6, **characterized in that** the angle (60) is different than 90°.

8. The instrument of claim 7, **characterized in that** the angle (60) between the longitudinal axis (58) of the shaft (12; 68), in the area of the distal end of the shaft (12; 68), and the plane (56) defined by the punch area lies between approximately 20° and approximately 80°.

9. The instrument of claim 7 or 8, **characterized in that** the angle (60) between the longitudinal axis (58) of the shaft (12; 68), in the area of the distal end of the shaft (12; 68), and the plane (56) defined by the punch area lies between approximately 30° and approximately 70°.

10. The instrument of anyone of claims 7 through 9, **characterized in that** the plane (56) defined by the punch area extends from top-proximal to bottom-distal.

11. The instrument of anyone of claims 7 through 9, **characterized in that** the plane (56) defined by the punch area extends from top-distal to bottom-proximal.

12. The instrument of anyone of claims 7 through 9, **characterized in that** the plane (56) defined by the punch area extends from right-proximal to left-distal.

13. The instrument of anyone of claims 7 through 9, **characterized in that** the plane (56) defined by the punch area extends from right-distal to left-proximal.

14. The instrument of anyone of claims 1 through 13, **characterized in that** the cutting element (19, 21; 78, 82) is designed about a complete circumference on the first or second tool (18, 20; 76, 80; 140, 142; 160, 170).

15. The instrument of anyone of claims 1 through 14, **characterized in that** the tools (18, 20; 76, 80; 140, 142; 160, 170) have an approximately circular cross section.

16. The instrument of claim 15, **characterized in that** the diameter of the tools (18, 20; 76, 80; 140, 142; 160, 170) lies in the range between approximately 2 and approximately 8 mm.

17. The instrument of claim 16, **characterized in that** the diameter of the tools (18, 20; 76, 80; 140, 142; 160, 170) lies in the range between approximately 3 mm and approximately 5 mm.

18. The instrument of anyone of claims 1 through 17, **characterized in that** the first tool (18; 76) and the second tool (20; 80) form a ball-shaped profile in the closed position.

19. The instrument of anyone of claims 1 through 18, **characterized in that** the first tool (18; 76) and the second tool (20; 80) form a double paraboloid or ellipsoid profile in the closed position.

20. The instrument of anyone of claims 1 through 19, **characterized in that** the first tool (18; 76) and the second tool (20; 80) form a tear-shaped profile in the closed position.

21. The instrument of anyone of claims 1 through 20, **characterized in that** the first tool (18; 76) and the second tool (20; 80) form a lobe-shaped profile in the closed position.

22. The instrument of anyone of claims 1 through 21, **characterized in that** one of the tools (18, 20; 76, 80; 140, 142; 160, 170) is movable and the second tool (18, 20; 76, 80; 140, 142; 160, 170) is immovable, and the immovable tool is arranged to the distal side of the movable tool.

23. The instrument of anyone of claims 1 through 22, **characterized in that** the first tool (18; 76; 140; 160) is secured against turning relative to the second tool (20; 80; 142; 170).

24. The instrument of anyone of claims 1 through 23, **characterized in that** both tools (18, 20; 76, 80; 140, 142; 160, 170) have cutting elements (19, 21; 78, 82).

25. The instrument of anyone of claims 1 through 24, **characterized in that** the shaft (12; 68) is straight.

26. The instrument of anyone of claims 1 through 25, **characterized in that** the shaft (12; 68) has at least one curve (44; 66).

27. The instrument of claim 26, **characterized in that** an angle of curvature of the curve (44; 66) lies in the range between approximately 20° and approximately 90°.

28. The instrument of claim 26 or 27, **characterized in that** an angle of curvature of the curve (44; 46) lies in the range between approximately 45° and approximately 65°.

## Revendications

1. Instrument médical (10 ; 62) pour la préparation de tissu humain et/ou animal, avec une tige (12 ; 68), avec un premier outil (18 ; 76 ; 140 ; 160) et un deuxième outil (20 ; 80 ; 142 ; 170) à une extrémité distale de la tige (12 ; 68), le premier outil (18 ; 76 ; 140 ; 160) et le deuxième outil (20 ; 80 ; 142 ; 170) coopérant grâce à un déplacement axial relatif l'un par rapport à l'autre à la manière d'une poinçonneuse et au moins l'un des outils (18, 20 ; 76, 80 ; 140, 142 ; 160, 170) comportant un élément coupant (19, 21 ; 78, 82) qui est disposé de manière à former avec l'autre outil (18, 20 ; 76, 80 ; 140, 142 ; 160, 170) une zone de poinçonnage qui définit un plan (56) qui forme un angle (60) non égal à 0° avec un axe longitudinal (50) de la tige (12 ; 68) dans la zone de l'extrémité distale de la tige (12 ; 68), le diamètre de l'outil du côté distal (18 ; 76 ; 140 ; 160) s'amincissant de manière convexe dans sa zone distale vers l'extrémité distale dans la direction longitudinale de la tige (12 ; 68), **caractérisé en ce que** les lignes de profil extérieur (111; 113 ; 144, 146) de l'outil du côté distal (18 ; 76 ; 140 ; 160), vues en coupe médiane longitudinale, se coupent à l'extrémité distale sous un angle α inférieur à 160° et **en ce que** l'outil du côté proximal (20 ; 80 ; 142 ; 170) présente un diamètre extérieur inférieur à une extrémité proximale de l'outil du côté distal (18 ; 76 ; 140 ; 160), de sorte que, lors du poinçonnage, l'outil du côté proximal (20 ; 80 ; 142 ; 170) s'enfonce dans l'outil du côté distal (18 ; 76 ; 140 ; 160).

2. Instrument selon la revendication 1, **caractérisé en ce que** l'angle (α) est inférieur à 140°.

3. Instrument selon la revendication 1 ou 2, **caractérisé en ce que** l'angle (α) est inférieur à 120°.

4. Instrument selon l'une quelconque des revendications 1 à 3, **caractérisé en ce que** l'angle (α) est inférieur à 100°.

5. Instrument selon l'une quelconque des revendications 1 à 4, **caractérisé en ce que** les lignes de profil extérieur (144, 146) se rejoignent pour former une pointe (148).

6. Instrument selon l'une quelconque des revendications 1 à 5, **caractérisé en ce que** la portion convexe de l'outil du côté distal (18 ; 76 ; 140 ; 160) se prolonge au moins approximativement par une portion droite s'étendant parallèlement à l'axe longitudinal de la tige (12).

7. Instrument selon l'une quelconque des revendications 1 à 6, **caractérisé en ce que** l'angle (60) n'est pas égal à 90°.

8. Instrument selon la revendication 7, **caractérisé en ce que** l'angle (60) entre l'axe longitudinal (58) de la tige (12 ; 68) dans la zone de l'extrémité distale de la tige (12 ; 68) et le plan (56) défini par la zone de poinçonnage est compris entre environ 20° et environ 80°.

9. Instrument selon la revendication 7 ou 8, **caractérisé en ce que** l'angle (60) entre l'axe longitudinal (58) de la tige (12 ; 68) dans la zone de l'extrémité distale de la tige (12 ; 68) et le plan (56) défini par la zone de poinçonnage est compris entre environ 30° et environ 70°.

10. Instrument selon l'une quelconque des revendications 7 à 9, **caractérisé en ce que** le plan (56) défini par la zone de poinçonnage s'étend entre le haut-proximal et le bas-distal.

11. Instrument selon l'une quelconque des revendications 7 à 9, **caractérisé en ce que** le plan (56) défini par la zone de poinçonnage s'étend entre le haut-distal et le bas-proximal.

12. Instrument selon l'une quelconque des revendications 7 à 9, **caractérisé en ce que** le plan (56) défini par la zone de poinçonnage s'étend entre la droite-proximale et la gauche-distale.

13. Instrument selon l'une quelconque des revendications 7 à 9, **caractérisé en ce que** le plan (56) défini par la zone de poinçonnage s'étend entre la droite-distale et la gauche-proximale.

14. Instrument selon l'une quelconque des revendications 1 à 13, **caractérisé en ce que** l'élément coupant (19, 21 ; 78, 82) est formé sur toute la circonférence sur le premier ou le deuxième outil (18, 20 ; 76, 80 ; 140, 142 ; 160, 170).

15. Instrument selon l'une quelconque des revendications 1 à 14, **caractérisé en ce que** les outils (18, 20 ; 76, 80 ; 140, 142 ; 160, 170) présentent une section transversale à peu près circulaire.

16. Instrument selon la revendication 15, **caractérisé en ce que** le diamètre des outils (18, 20 ; 76, 80 ; 140, 142 ; 160, 170) se trouve dans la plage comprise entre environ 2 mm et environ 8 mm.

17. Instrument selon la revendication 16, **caractérisé en ce que** le diamètre des outils (18, 20 ; 76, 80 ; 140, 142 ; 160, 170) se trouve dans la plage comprise entre environ 3 mm et environ 5 mm.

18. lnstrument selon l'une quelconque des revendications 1 à 17, **caractérisé en ce que** le premier outil (18 ; 76) et le deuxième outil (20 ; 80) forment un profil sphérique en position de fermeture.

19. Instrument selon l'une quelconque des revendications 1 à 18, **caractérisé en ce que** le premier outil (18 ; 76) et le deuxième outil (20 ; 80) forment un profil bi-paraboloïdal ou ellipsoïdal en position de fermeture.

20. Instrument selon l'une quelconque des revendications 1 à 19, **caractérisé en ce que** le premier outil (18 ; 76) et le deuxième outil (20 ; 80) forment un profil en forme de goutte en position de fermeture.

21. Instrument selon l'une quelconque des revendications 1 à 20, **caractérisé en ce que** le premier outil (18 ; 76) et le deuxième outil (20 ; 80) forment un profil en forme de lobe en position de fermeture.

22. Instrument selon l'une quelconque des revendications 1 à 21, **caractérisé en ce que** l'un des outils (18, 20 76, 80 ; 140, 142 ; 160, 170) est mobile et le deuxième outil (18, 20 ; 76, 80 ; 140, 142 ; 160, 170) est fixe et **en ce que** l'outil fixe est disposé du côté distal de l'outil mobile.

23. Instrument selon l'une quelconque des revendications 1 à 22, **caractérisé en ce que** le premier outil (18 ; 76 ; 140 ; 160) est bloqué contre la rotation par rapport au deuxième outil (20 ; 80 ; 142 ; 170).

24. lnstrument selon l'une quelconque des revendications 1 à 23, **caractérisé en ce que** les deux outils (18, 20 ; 76, 80 ; 140, 142; 160, 170) comportent des éléments coupants (19, 21 ; 78, 82).

25. Instrument selon l'une quelconque des revendications 1 à 24, **caractérisé en ce que** la tige (12 ; 68) est droite.

26. Instrument selon l'une quelconque des revendications 1 à 25, **caractérisé en ce que** la tige (12 ; 68) présente au moins une courbure (44 ; 66).

27. Instrument selon la revendication 26, **caractérisé en ce qu'**un angle de courbure de la courbure (44 ; 66) se trouve dans la plage comprise entre environ 20° et environ 90°.

28. Instrument selon la revendication 26 ou 27, **caractérisé en ce qu'**un angle de courbure de la courbure (44 ; 66) se trouve dans la plage comprise entre environ 45° et environ 65°.
